# EUROPEAN PATENT APPLICATION

(11) **EP 1 849 757 A1**
(43) Date of publication of application: **31.10.2007**
(21) Application number: 06713681.2
(22) Date of filing: 14.02.2006
(51) Int. Cl.: C07C 2/34, C07C 11/02, C07B 61/00

(54) **METHOD FOR PRODUCING UNSATURATED HYDROCARBON COMPOUND**

(30) Priority: 18.02.2005 JP 2005042765
(71) Applicant: IDEMITSU KOSAN CO., LTD., Tokyo 100-8321 (JP)
(72) Inventor: FUJIKAWA, Shinjiro, Chiba, 2990107 (JP); OKAMOTO, Takuji, Chiba, 2990107 (JP); YOKOTA, Kiyohiko, Chiba, 2990107 (JP)
(74) Representative: Vossius & Partner
(86) International application number: PCT/JP2006/302540
(87) International publication number: WO 2006/088020

(57) **Abstract**

Disclosed is a method for producing an unsaturated hydrocarbon compound wherein an α-olefin is dimerized by using a catalyst system composed of a metallocene compound (A), an ionizing agent (B) and hydrogen. By this method, an unsaturated hydrocarbon compound having unsaturated double bonds in a high ratio, in particular the one having a terminal vinylidene group can be produced efficiently.

## Description

### Technical Field

The present invention relates to an efficient method for producing an unsaturated hydrocarbon compound useful as a lubricant, cleaning agent, additive, its intermediate or the like.

### Background Art

Dimerization reaction of an α-olefin using a catalyst composed of a combination of a metallocene, an aluminoxane, an organoaluminum compound and the like is publicly known, and examples of such catalyst include a catalyst composed of zirconocene and methylaluminoxane (for example, Patent Document 1), a catalyst composed of zirconocene, organoaluminum and borate (for example, Patent Document 2), a catalyst composed of zirconocene, alkyl aluminoxane and trimethylaluminum (for example, Patent Document 3), and others.
Moreover, oligomerization of an α-olefin is also publicly known, for which a catalyst composed of zirconocene, organoaluminum and borate (for example, Patent Document 4), a catalyst composed of zirconocene, organoaluminum and borane (for example, Patent Document 5) and the like are used.
However, as activity of these catalyst systems are low and a large amount of the catalyst is required, productivity is inferior and, therefore, industrial utilization is difficult.

Furthermore, addition of hydrogen to dimerization and oligomerization reaction systems is not known.
In a polymerization reaction using a metallocene catalyst, hydrogen is added to control the molecular weight of the polymer.
This is to cause a chain transfer reaction by the addition of hydrogen, and as a result, terminal groups of the polymer are saturated (for example, Patent Documents 6 and 7).
Therefore, to obtain a polymer having unsaturated double bonds in a high ratio, the addition of hydrogen has been considered to be avoided.

Patent Document 1: Japanese Examined Patent Publication No. H07-116065
Patent Document 2: Japanese Patent No. 2752538
Patent Document 3: Japanese Patent No. 3073234
Patent Document 4: Japanese Publication of Translation of PCT No. 2002-518582
Patent Document 5: Japanese Publication of Translation of PCT No. 2002-522572Patent Document 6: Japanese Patent No. 3058419
Patent Document 7: Japanese Patent No. 3086469

### Disclosure of the Invention

The present invention is to address the above problems, and an object of the present invention is to provide a method for efficiently producing an unsaturated hydrocarbon compound having unsaturated double bonds in a high ratio, particularly a terminal vinylidene group (α-olefin dimer).

The reaction between a metallocene catalyst and an α-olefin normally proceeds by a 1,2-insertion reaction, but if a 2,1-insertion reaction takes place at a certain probability, the reaction does not proceed and the catalyst enters into a dormant state.
Although this dormant catalyst does not react with the α-olefin, it reacts with hydrogen and an active catalyst is regenerated.
The inventors of the present invention have found that, by limiting the chain transfer reaction caused by hydrogen as far as possible, and by adding an amount of hydrogen necessary for the regeneration of the dormant catalyst, an unsaturated hydrocarbon compound having unsaturated terminal double bonds in a high ratio is obtained, and thus accomplished the present invention. The present invention was completed based on such knowledge.

Namely, the present invention provides the following:
1. A method for producing an unsaturated hydrocarbon compound, wherein an α-olefin is dimerized by using a catalyst composed of a metallocene compound (A) and an ionizing agent (B) in the presence of hydrogen,
2. The method for producing an unsaturated hydrocarbon compound described in the above 1, wherein the catalyst contains an organoaluminum compound (C) in addition to the component (A) and the component (B),
3. The method for producing unsaturated hydrocarbon compound described in the above 1 or 2, wherein the ionizing agent (B) is an oxygen-containing organometallic compound (b-1),
4. The method for producing an unsaturated hydrocarbon compound described in the above 3, wherein the component (b-1) is represented by the general formula (2) and/or the general formula (3), 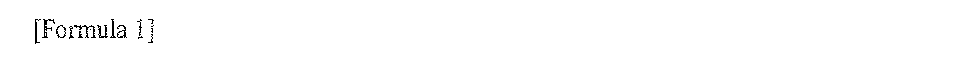 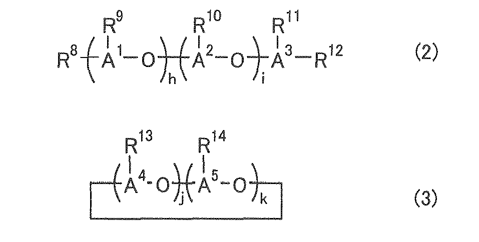 in the formula, R⁸ to R¹⁴ each independently represent an alkyl group having 1 to 8 carbon atoms, A¹ to A⁵ each independently represent a metal element of Group 13 in the periodic table. Each of h to k is a number of 0 to 50, and both (h + i) and (j + k) are larger than 1,
5. The method for producing an unsaturated hydrocarbon compound described in the above 4,
   wherein A¹ to A⁵ are aluminum.
6. The method for producing an unsaturated hydrocarbon compound described in the above 1 or 2, wherein the ionizing agent (B) is a compound (b-2) forming an ionic complex by reacting with the metallocene compound (A),
7. The method for producing an unsaturated hydrocarbon compound described in the above 6, wherein the compound forming an ionic complex by reacting with the metallocene compound (A) is a coordination complex composed of an anion and a cation wherein plural groups are bonded to a metal and/or a Lewis acid,
8. The method for producing an unsaturated hydrocarbon compound described in the above 7, wherein the coordination complex composed of an anion and a cation wherein plural groups are bonded to the metal and/or the Lewis acid is represented by the general formula (4) and/or (5),

   ([L¹ - H]^{g+})_{f}([M²D¹D² ... Dⁿ]^{(n-m)-})₁ (4)

   ([L²]^{g+})_{f}([M³D¹D² ... Dⁿ]^{(n-m)-})₁ (5)

   in the formula, L¹ represents a Lewis base, L² represents M⁴, R¹⁵R¹⁶M⁵ or R¹⁷₃C, M² and M³ each represent a metal selected from Group 5 to Group 15 in the periodic table, M⁴ represents a metal selected from Group 1 and Group 8 to Group 12 in the periodic table, M⁵ represents a metal selected from Group 8 to Group 10 in the periodic table, D¹ to Dⁿ each represent a hydrogen atom, a dialkylamino group, an alkoxy group, an aryloxy group, an alkyl group having 1 to 20 carbon atoms, an aryl group having 6 to 20 carbon atoms, an alkylaryl group, an arylalkyl group, a substituted alkyl group, an organometalloid group or a halogen atom. R¹⁵ and R¹⁶ each represent a cyclopentadienyl group, a substituted cyclopentadienyl group, an indenyl group or a fluorenyl group, R¹⁷ represents an alkyl group having 1 to 20 carbon atoms, an aryl group having 6 to 20 carbon atoms or an alkylaryl group. The symbol m is the valence of M² and M³, and is an integer of 1 to 7, n is an integer of 2 to 8, g is the ionic valence of [L¹ - H] and [L²] and is an integer of 1 to 7, f is an integer of 1 or larger, and 1 is a value calculated from the formula [f×g/(n - m)].
9. The method for producing an unsaturated hydrocarbon compound described in the above 8, wherein M² and M³ represent boron,
10. A method for producing an unsaturated hydrocarbon compound described in any of the above 1 to 9, wherein the component (A) is a compound represented by the general formula (1),

   Qₐ(C₅H_{5-a-b}R¹_{b})(C₅H_{5-a-c}R²_{c})M¹XY (1)

   in the formula, Q represents a connecting group for crosslinking two conjugated 5-membered ring ligands (C₅H_{5-a-b}R¹_{b}) and (C₅H_{5-a-c}R²_{c}). R¹ and R² each represent a hydrocarbon group, a halogen atom, an alkoxy group, a silicon-containing hydrocarbon group, a phosphorus-containing hydrocarbon group, a nitrogen-containing hydrocarbon group or a boron-containing hydrocarbon group, and may be the same or different from each other when plural groups are present, or may form a ring structure by bonding with each other. The symbol a is 0, 1 or 2. The symbols b and c each are an integer of 0 to 5 when a = 0, an integer of 0 to 4 when a = 1, and an integer of 0 to 3 when a = 2. M¹ represents a transition metal of Group 4 in the periodic table. X and Y each represent a covalent bond ligand or an ionic bond ligand, and may be bonded with each other.
11. The method for producing an unsaturated hydrocarbon compound described in the above 10, wherein M¹ represents zirconium.
12. The method for producing an unsaturated hydrocarbon compound described in any of the above 1 to 11, wherein the ratio of unsaturated double bonds contained in the unsaturated hydrocarbon compound is 80 mol % or more.

According to the present invention, an unsaturated hydrocarbon compound having unsaturated double bonds in a high ratio, particularly a terminal vinylidene group may be economically produced in a high yield and high selectivity by using a catalyst system containing a metallocene compound (A) and an ionizing agent (B) and by controlling the reaction of hydrogen addition.

### [Best Mode for Carrying Out the Invention]

The present invention relates to a method for producing an unsaturated hydrocarbon compound by dimerization of an α-olefin using a catalyst composed of a metallocene compound (A), an ionizing agent (B) and, if necessary, an organoaluminum compound (C) in the presence of hydrogen.
The following compounds may be preferably used as a component of the catalyst related to the present invention.

Metallocene compound (A)
In the present invention, although various metallocene compounds may be used, preferably a transition metal compound of Group 4 in the periodic table may be used.
The transition metal compound of Group 4 in the periodic table is represented by the general formula (1),

Qₐ(C₅H_{5-a-b}R¹_{b})(C₅H_{5-a-c}R²_{c})M¹XY (1)

where Q represents a connecting group for crosslinking two conjugated 5-membered ring ligands (C₅H_{5-a-b}R¹_{b}) and (C₅H_{5-a-c}R²_{c}). R¹ and R² each represent a hydrocarbon group, a halogen atom, an alkoxy group, a silicon-containing hydrocarbon group, a phosphorus-containing hydrocarbon group, a nitrogen-containing hydrocarbon group or a boron-containing hydrocarbon group, and may be the same or different from each other when plural groups are present, or may form a ring structure by bonding with each other. The symbol a is 0, 1 or 2. The symbols b and c each are an integer of 0 to 5 when a = 0, an integer of 0 to 4 when a = 1, and an integer of 0 to 3 when a = 2. M¹ represents a transition metal of Group 4 in the periodic table. X and Y each represent a covalent bond ligand or an ionic bond ligand, and may be bonded with each other.
Specific examples of Q include:
(1) Alkylene group having 1 to 20 carbon atoms such as methylene group, ethylene group, propylene group, butylenes group, isopropylene group, methylphenyl methylene group, diphenyl methylene group, cyclohexylene group and the like, cycloalkylene group, or cycloalkylene group having side chain lower alkyl or phenyl-substituted cycloalkylene group.
(2) Silylene group such as silylene group, dimethylsilylene group, methylphenyl silylene group, diphenylsilylene group, disilylene group, tetramethyldisilylene group and the like, oligo silylene group, or oligo silylene group having side chain lower alkyl or phenyl-substituted oligo silylene group.
(3) Hydrocarbon group containing germanium, phosphorus, nitrogen, boron or aluminum [lower alkyl group, phenyl group, hydrocarbyloxy group (preferably lower alkoxy group)] such as (CH₃)₂Ge group, (C₆H₅)₂Ge group, (CH₃)P group, (C₆H₅)P group, (C₄H₉)N group, (C₆H₅)N group, (CH₃)B group, (C₄H₉)B group, (C₆H₅)B group, [(i-C₃H₇)₂N]B group, (C₆H₅)Al group, (CH₃O)Al group, and the like.
Among them, alkylene group and silylene group are preferable.

Furthermore, (C₅H_{5-a-b}R¹_{b})and (C₅H_{5-a-c}R²_{c}) each represent a conjugated 5-membered ring ligand. R¹ and R² each represent a hydrocarbon group, a halogen atom, an alkoxy group, a silicon-containing hydrocarbon group, a phosphorus-containing hydrocarbon group, a nitrogen-containing hydrocarbon group or a boron-containing hydrocarbon group. The symbol a is 0, 1 or 2.
The symbols b and c each are an integer of 0 to 5 when a = 0, an integer of 0 to 4 when a = 1, and an integer of 0 to 3 when a = 2.
As the hydrocarbon group mentioned above, those having 1 to 20 carbon atoms are preferable, and those having 1 to 12 carbon atoms are particularly preferable.
The hydrocarbon group as a monovalent group may be bonded to a cyclopentadienyl group that is a conjugated 5-membered ring group, or when plural groups are present, two of them may be bonded with each other to form a ring structure together with a part of the cyclopentadienyl group.
Namely, representative examples of the conjugated 5-membered ring ligand include substituted or unsubstituted cyclopentadienyl group, indenyl group and fluorenyl group.
As the halogen atom, chlorine, bromine, iodine and fluorine atoms are cited, and as alkoxy group, those having 1 to 12 carbon atoms are preferably cited .
Examples of the silicon-containing hydrocarbon group include -Si(R³)(R⁴)(R⁵) (where R³, R⁴ and R⁵ each represent a hydrocarbon group having 1 to 24 carbon atoms) and the like, examples of the phosphorous-containing hydrocarbon group, nitrogen-containing hydrocarbon group and boron-containing hydrocarbon group include, respectively, -P(R⁶)(R⁷), -N(R⁶)(R⁷), -B(R⁶)(R⁷) and the like, where R⁶ and R⁷ each represent a hydrocarbon group having 1 to 18 carbon atoms.
When plural R¹s and R²s are present, they may be the same or different from each other.
Furthermore, in a compound represented by the general formula (1), conjugated 5-membered ring ligands (C₅H_{5-a-b}R¹_{b}) and (C₅H_{5-a-c}R²_{c}) may be the same or different from each other.

M¹ represents a transition metal element of Group 4 in the periodic table, and specific examples include titanium, zirconium, hafnium, of which zirconium is most preferable.
X and Y each represent a covalent bonding ligand or an ionic bonding ligand. Specific examples include hydrogen atom, halogen atom, hydrocarbon group having 1 to 20 carbon atoms, preferably 1 to 10 carbon atoms, alkoxy group having 1 to 20 carbon atoms, preferably 1 to 10 carbon atoms, amino group, phosphorous-containing hydrocarbon group having 1 to 20 carbon atoms, preferably 1 to 12 carbon atoms (such as diphenyl phosphine group), silicon-containing hydrocarbon group having 1 to 20 carbon atoms, preferably 1 to 12 carbon atoms (such as trimethylsilyl group), hydrocarbon group having 1 to 20 carbon atoms, preferably 1 to 12 carbon atoms, or halogen-containing boron compound (such as B(C₆H₅)₄, BF₄).
Among those groups, hydrogen atom, halogen atom, hydrocarbon group and alkoxy group are preferable.
X and Y may be the same or different from each other.

Specific examples of a compound represented by the above general formula (1) include compounds described in the following (a) to (f):
(a) Transition metal compound having two conjugated 5-membered ring ligands without having a crosslinking bonding group, such as bis(cyclopentadienyl)zirconium dichloride, bis(methylcyclopentadienyl)zirconium dichloride, bis(1,3-dimethylcyclopentadienyl) zirconium dichloride, bis(1,2,4-trimethylcyclopentadienyl)zirconium dichloride, bis(tetramethylcyclopentadienyl)zirconium dichloride, bis(pentamethyl cyclopentadienyl)zirconium dichloride, bis(n-butylcyclopentadienyl)zirconium dichloride, bis(trimethylsilylcyclopentadienyl)zirconium dichloride, bis[bis(trimethylsilyl)cyclopentadienyl]zirconium dichloride, bis(trimethylsilylmethyl cyclopentadienyl)zirconium dichloride, bis(indenyl)zirconium dichloride, bis(1,2,3-trimethylindenyl)zirconium dichloride, bis(1,2,3-trimethyltetrahydro indenyl)zirconium dichloride, bis(fluorenyl)zirconium dichloride, bis(9-methylfluorenyl)zirconium dichloride, bis(9-methyloctahydrofluorenyl)zirconium dichloride, bis(cyclopentadienyl)zirconium chlorohydride, bis(cyclopentadienyl)methyl zirconium chloride, bis(cyclopentadienyl)ethyl zirconium chloride, bis(cyclopentadienyl)methoxy zirconium chloride, bis(cyclopentadienyl)phenyl zirconium chloride, bis(cyclopentadienyl)dimethyl zirconium, bis(cyclopentadienyl)diphenyl zirconium, bis(cyclopentadienyl)dineopentyl zirconium, bis(cyclopentadienyl)dihydrozirconium, bis(cyclopentadienyl)dimethoxyzirconium, (cyclopentadienyl)(pentamethylcyclopentadienyl)zirconium dichloride, (cyclopentadienyl)(indenyl)zirconium dichloride, (cyclopentadienyl)(fluorenyl) zirconium dichloride, and the like,

(b) Transition metal compound having two conjugated 5-membered ring ligands crosslinked by an alkylene group such as methylenebis(cyclopentadienyl)zirconium dichloride, ethylenebis(cyclopentadienyl)zirconium dichloride, 1,3-propylene bis(cyclopentadienyl)zirconium dichloride, 1,4-butylenebis(cyclopentadienyl) zirconium dichloride, isopropylidenebis(cyclopentadienyl)zirconim dichloride, rac-methylenebis(indenyl)ziroconium dichloride, meso-methylenebis(indenyl) zirconium dichloride, rac-ethylenebis(indenyl)zirconium dichloride, meso-ethylene bis(indenyl)zirconium dichloride, ethylenebis(fluorenyl)zirconium dichloride, rac-1,3-prpopylenebis(indenyl)zirconium dichloride, meso-1,3-propylene bis(indenyl)zirconium dichloride, rac-1,4-butylenebis(indenyl)zirconium dichloride, meso-1,4-butylenebis(indenyl)zirconium dichloride, rac-ethylene bis(4,5,6,7-tetrahydro indenyl)zirconium dichloride, meso-ethylenebis(4,5,6,7-tetrahydroindenyl)zirconium dichloride, rac-ethylenebis(2-methylmdenyl)zirconium dichloride, meso-ethylene bis(2-methylindenyl)zirconium dichloride, rac-ethylenebis(2,3-dimehylindenyl) zirconium dichloride, meso-ethylenebis(2,3-dimethylindenyl)zirconium dichloride, ethylene(2,4-dimethylcyclopentadienyl)(3',5'-dimethylcyclopentadienyl)zirconium dichloride, ethylene(2-methyl-4-tert-butylcyclopentadienyl)(3'-tert-butyl-5'-methyl cyclopentadienyl)zirconium dichloride, ethylene(2,3,5-trimethylcyclopentadienyl) (2',4',5'-trimethylcyclopentadienyl)zirconium dichloride, ethylenebis(tetramethyl cyclopentadienyl)zirconium dichloride, methylene(cyclopentadienyl)(3,4-dimethyl cyclopentadienyl)zirconium dichloride, methylene(cyclopentadienyl)(trimethyl cyclopentadienyl)zirconium dichloride, methylene(cyclopentadienyl)(tetramethyl cyclopentadienyl)zirconium dichloride, isopropylidene(cyclopentadienyl) (3,4-dimethylcyclopentadienyl)zirconium dichloride, isopropylidene(cyclopentadienyl) (tetramethylcyclopentadienyl)zirconium dichloride, isopropylidene(cyclopentadienyl) (3-methylindenyl)zirconium dichloride, isopropylidene(cyclopentadienyl)(fluoreyl) zirconium dichloride, isopropylidene(2-methylcyclopentadienyl)(fluorenyl)zirconium dichloride, isopropylidene(2,5-dimethylcyclopentadienyl) (3,4-dimethylcyclopentadienyl) zirconium dichloride, isopropylidene(2,5-dimethylcyclopentadienyl)(fluorenyl)zirconium dichloride, ethylene(cyclopentadienyl)(2,4-dimethylcyclopentadienyl)zirconium dichloride, ethylene(cyclopentadienyl)(flurenyl)zirconium dichloride, ethylene(2,5-dimethyl cyclopentadienyl)(fluorenyl)zirconium dichloride, ethylene(2,5-diethyl cyclopentadienyl)(fluorenyl)zirconium dichloride, diphenylmethylene (cyclopentadienyl)(3,4-diethylcyclopentadienyl)zirconium dichloride, diphenyl methylene(cyclopentadienyl)(3,4-diethylopentadienyl)zirconium dichloride, cyclohexylidene(cyclopentadienyl)(fluorenyl)zirconium dichloride, cyclohexylidene (2,5-dimethylcyclopentadienyl)(3',4' -dimethylcyclopentadienyl)zirconium dichloride, and the like,

(c) Transition metal compound having two conjugated 5-membered ring ligands crosslinked by a silylene group such as dimethylsilylene bis(cyclopentadienyl)zirconium dichloride, phenylmethylsilylene bis(cyclopentadienyl)zirconium dichloride, diphenylsilylene bis(cyclopentadienyl)zirconium dichloride, tetramethyldisilylene bis(cyclopentadienyl)zirconium dichloride, dimethylsilylene(cyclopentadienyl) (tetramethylcyclopentadienyl)zirconium dichloride, and the like,
(d) Transition metal compound having two conjugated 5-membered ring ligands crosslinked by a hydrocarbon group containing germanium, aluminum, boron, phosphorous or nitrogen such as dimethylgermanediylbis(cyclopentadienyl)zirconium dichloride, methylaluminumdiylbis(cyclopentadienyl)zirconium dichloride, phenylaluminumdiylbis(cyclopentadienyl)zirconium dichloride, phenylphosphinediyl bis(cyclopentadienyl)zirconium dichloride, ethylboranediyl bis(cyclopentadienyl) zirconium dichloride, diisopropylaminoboranediylbis(cyclopentadienyl)zirconium chloride, phenylaminediylbis(cyclopentadienyl)zirconium dichloride, and the like,
(e) Transition metal compound having two conjugated 5-membered ring ligands doubly crosslinked with each other such as (1,1'-dirnethylsilylene)-(2,2'-isopropylidene) bis(cyclopentadienyl)zirconium dichloride, (1,1'-ethylene) (2,2'-ethylene) bis(cyclopentadienyl)zirconium dichloride, (1,1'-dimethylsilylene) (2,2'-dimethylsilylene)bis(cyclopentadienyl)zirconium dichloride, (1,2'-dimethylsilylene)(2,1'-ethylene)bis(indenyl)zirconium dichloride, (1,1'-dimethylsilylene) (2,2'-ethylenc)bis(indenyl)zirconium dichloride, (1,1'-ethylene) (2,2'-dimethylsilylene) bis(indenyl)zirconium dichloride, (1,1'-dimethylsilylene)(2,2'-cyclohexylidene) bis(indenyl)zirconium dichloride, and the like,
(f) Furthermore, a compound described in the above (a) to (e), wherein chlorine atom is replaced with bromine atom, iodine atom, hydrogen atom, methyl group, phenyl group and the like, and the centered metal of the above compounds, zirconium, is replaced with titanium or hafnium.

Ionizing agent (B)
In the present invention, the oxygen-containing organometallic compound (b-1) and the compound (b-2) forming an ionic complex by reacting with the metallocene compound (A) described above may be used as an ionizing agent.
The oxygen-containing organometallic compound (b-1) represented by the general formula (2) and/or the general formula (3) is preferably used, 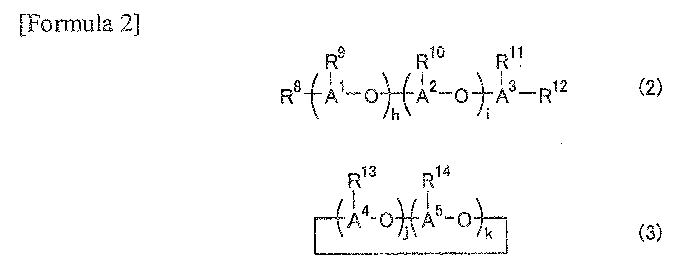 where R⁸ to R¹⁴ each independently represent an alkyl group having 1 to 8 carbon atoms, A¹ to A⁵ each independently represent a metal element of Group 13 in the periodic table. The symbols h to k each are an integer of 0 to 50, and both (h + i) and (j + k) are 1 or larger.
In the compounds represented by the general formulas (2) and (3), alkyl groups represented by R⁸ to R¹⁴ include methyl group, ethyl group, n-propyl group, iso-propyl group, various butyl groups, various pentyl groups, various hexyl groups, various heptyl groups, and various octyl groups. The metal elements represented by A¹ to A⁵ belonging to Group 13 metal elements in the periodic table may include boron, aluminum, gallium, indium, and thallium.
Among those metal elements, boron and aluminum are particularly preferable. Further, the range of h to k is preferably in the range of 1 to 20, and particularly 1 to 5.
The compounds represented by the general formulas (2) and (3) include alumoxanes such as straight chain or cyclic tetramethyl dialumoxane, tetraisobutyldialumoxane, methyl alumoxane, ethyl alumoxane, butyl alumoxane, isobutylalumoxane and the like; boroxanes such as trimethyl boroxane, methyl boroxane and the like.
Among those compounds, alumoxanes are preferable, and particularly methyl alumoxane are preferable.

(b-2) The compound forming an ionic complex by reacting with the metallocene compound (A) includes a coordination complex composed of an anion and a cation wherein plural groups are bonded to a metal and/or a Lewis acid.
As the coordination complex composed of an anion and a cation wherein plural groups are bonded to a metal mentioned above, a compound represented by the general formula (4) and/or (5) is preferable,

([L¹ - H]^{g+})_{f}([M²D¹D² ... Dⁿ]^{(n-m)-})₁ (4)

([L²]^{g+})_{f}([M³D¹D² ... Dⁿ]^{(n-m)-})₁ (5)

where L¹ represents a Lewis base, L² represents M⁴, R¹⁵R¹⁶M⁵ or R¹⁷₃C, M² and M³ each represent a metal selected from Group 5 to Group 15 in the periodic table, M⁴ represents a metal selected from Group 8 to Group 12 in the periodic table, M⁵ is a metal selected from Group 8 to 1 Group 10 in the periodic table, D¹ to Dⁿ each represent a hydrogen atom, a dialkylamino group, an alkoxy group, an aryloxy group, an alkyl group having 1 to 20 carbon atoms, an aryl group having 6 to 20 carbon atoms, an alkylaryl group, an arylalkyl group, a substituted alkyl group, an organometalloid group or a halogen atom. R¹⁵ and R¹⁶ each represent a cyclopentadienyl group, a substituted cyclopentadienyl group, an indenyl group or a fluorenyl group, R¹⁷ represents an alkyl group having 1 to 20 carbon atoms, an aryl group having 6 to 20 carbon atoms or an alkylaryl group. The symbol m is the valence of M², M³ and is an integer of 1 to 7, n is an integer of 2 to 8, g is the ionic valence of [L¹ - H] and [L²], and is an integer of 1 to 7, f is an integer of 1 or larger, 1 is a value calculated from the formula [f× g/(n - m)].

As the metal represented by M² and M³ in the general formulas (4) and (5), boron, aluminum, silicon, phosphorous, arsenic, antimony are preferable, as the metal represented by M⁴, silver, cupper, sodium, lithium are preferable, and as the metal represented by M⁵, iron, cobalt, nickel and the like are preferable.
Specific examples of D¹ to Dⁿ in the compound represented by the general formulas (4) and (5) include dialkyl amino group such as dimethyl amino group, diethyl amino group and the like, alkoxy group such as methoxy group, ethoxy group, n-butoxy group and the like, aryloxy group such as phenoxy group, 2,6-dimethylphenoxy group, naphthyloxy group and the like.
As the alkyl group having 1 to 20 carbon atoms, methyl group, ethyl group, n-propyl group, isopropyl group, n-butyl group, n-octyl group, 2-ethylhexyl group and the like are preferable. As the aryl group, alkylaryl group or arylalkyl group having 6 to 20 carbon atoms, phenyl group, p-tolyl group, benzyl group, pentafluorophenyl group, 3,5-di(trifluoromethyl)phenyl group, 4-tert-butylphenyl group, 2,6-dimethylphenyl group, 3,5-dimethylphenyl group, 2,4-dimethylphenyl group, 2,3-dimethylphenyl group and the like are preferable.
Furthermore, as the halogen atom, fluorine atom, chlorine atom, bromine atom, and iodine atom are preferable, and as the organometalloid group, pentamethylantimony group, trimethylsilyl group, trimethylgermyl group, diphenylarsine group, dicyclohexyl antimony group, diphenylboron group and the like are preferable.
Also, as the substituted cyclopentadienyl group represented by R¹⁵ and R¹⁶, methylcyclopentadienyl group, butylcyclopentadienyl group, pentamethyl cyclopentadienyl group and the like are preferable.

As the anion of the present invention in which plural groups are bonded to a metal, preferable are specifically B(C₆F₅)4⁻, B(C₆HF₄)₄⁻, B(C₆H₂F₃)₄⁻, B(C₆H₃F₂)₄⁻, B(C₆H₄F)₄⁻, B[C₆(CF₃)F₄]₄⁻, B(C₆H₅)₄⁻, FB(C₆F₅)₃⁻, FB(C₁₀F₇)₃⁻, PF₆⁻, P(C₆F₅)₆⁻, Al(C₆F₅)₄⁻, Al(C₆HF₄)₄⁻, FAl(C₆F₅)₃⁻, FAl(C₁₀F₇)₃⁻, and the like.
As the metal cation, preferable are CP₂Fe⁺, (MeCp)₂Fe⁺, (tBuCp)₂Fe⁺, (Me₂Cp)₂Fe⁺, (Me₃Cp)₂Fe⁺, (Me₄Cp)₂Fe⁺, (Me₅Cp)₂Fe⁺, Ag⁺, Na⁺, Li⁺ and the like.
Further, examples of other cations include a nitrogen-containing compound such as pyridinium, 2,4-dinitro-N,N-diethylanilinium, diphenylammonium, p-nitroanilinium, 2,5-dichloroanilinium, p-nitro-N,N-dimethylanilimium, quinolinium, N,N-dimethylanilinium, N,N-diethylanilinium and the like, a carbenuim compound such as triphenylcarbenium, tri(4-methylphenyl)carbenium, tri(4-methoxyphenyl)carbenium and the like, alkylphosphonium ion such as CH₃PH₃⁺, C₂H₅PH₃⁺, C₃H₇PH₃⁺, (CH₃)₂PH₂⁺, (C₃H₅)₂PH₂⁺, (C₃H₇)₂PH₂⁺, (CH₃)₃PH⁺, (C₂H₅)₃PH⁺, (C₃H₇)₃PH⁺, (CF₃)₃PH⁺, (CH₃)₄P⁺, (C₂H₅)₄P⁺, (C₃H₇)₄P⁺ and the like, and arylphosphonium ion such as C₆H₅PH₃⁺, (C₆H₅)₂PH₂⁺, (C₆H₅)₃PH⁺, (C₆H₅)₄P⁺, (C₂H₅)₂(C₆H₅)PH⁺, (CH₃)(C₆H₅)PH₂⁺, (CH₃)₂(C₆H₅)PH⁺ (C₂H₅)₂(C₆H₅)₂P⁺ and the like.

Preferable examples of the compound represented by the general formula (4) include triethylammonium tetraphenylborate, tri(n-butyl)ammonium tetraphenylborate, trimethylammonium tetraphenylborate, triethylammonium tetrakis(pentafluorophenyl)borate, tri(n-butyl)ammonium tetrakis(pentafluorophenyl)borate, triethylammonium hexafluoroarsenate, pyridinium tetrakis(pentafluorophenyl)borate, pyrrolinium tetrakis(pentafluorophenyl)borate, N,N-dimethylanilinium tetrakis(pentafluoro-phenyl)borate, methyldiphenylammonium tetrakis(pentafluorophenyl)borate and the like.
Preferable examples of the compound represented by the general formula (5) include ferrocenium tetraphenylborate, ferrocenium tetrakis(pentafluorophenyl)borate, dimethylferrocenium tetrakis(pentafluorophenyl)borate, decamethylferrocenium tetrakis(pentafluorophenyl)borate, acetylferrocenium tetrakis(pentafluorophenyl)borate, formylferrocenium tetrakis(pentafluorophenl)borate, cyanoferrocenium tetrakis (pentafluorophenyl)borate, silver tetraphenylborate, silver tetrakis(pentafluorophenyl) borate, trityl tetraphenylborate, trityl tetrakis(pentafluorophenyl)borate, silver hexafluoroarsenate, silver hexafluoroantimonate, silver tetrafluoroborate and the like. Furthermore, preferable examples of the Lewis acid include B(C₆F₅)₃, B(C₆HF₄)₃, B(C₆H₂F₃)₃, B(C₆H₃F₂)₃, B(C₆H₄F)₃, B(C₆H₅)₃, BF₃, B[C₆(CF₃)F₄]₃, B(C₁₀F₇)₃, FB(C₆F₅)₂, PF₅, P(C₆F₅)₅, Al(C₆F₅)₃, Al(C₆HF₄)₃, Al(C₁₀F₇)₃ and the like.

When using the ionizing agent (B), namely, the oxygen-containing organometallic compound (b-1) and the compound (b-2) capable of forming an ionic complex by reacting with a transition metal compound as a component of the catalyst, the component (b-1) may be used alone, or in a combination of two or more.
Moreover, the component (b-2) may be also used alone or in a combination of two or more.
Furthermore, the component (b-1) and the component (b-2) may be used suitably in combination with each other.

(C) Organoaluminum compound
The organoaluminum compound preferably used in the present invention is represented by the general formula (6),

R¹⁸ ₚAL(OR¹⁹)_{q}H_{3-p-q} (6)

where R¹⁸ and R¹⁹ each independently represent a hydrocarbyl group or an organometalloid group each having 1 to 20 carbon atoms. The symbol p is 0<p≦3, and q is 0≦q<3.
In the compound represented by the general formula (6), preferable examples of the hydrocarbyl group of R¹⁸ and R¹⁹ include alkyl group such as methyl group, ethyl group, propyl group, isopropyl group, various butyl group and the like, aralkyl group such as benzyl group, triphenylmethyl group and the like, aryl group such as phenyl group, substituted phenyl group and the like, and various hydrocarbyl silyl groups.
The symbol p is preferably 2 or 3 and more preferably 3.
The symbol q is preferably 0 or 1.
Examples of the compound represented by the general formula (6) include trialkylaluminum such as trimethylaluminum, triethylaluminum, tri-n-propylaluminum, triisopropylaluminum, tri-n-butylaluminum, triisobutylaluminum, tri-tert-butylaluminum and the like, dialkylaluminumalkoxide such as dimethyl aluminummethoxide, diethylaluminummethoxide, diethylaluminumethoxide, dimethyl aluminum(2,6-diisopropylphenoxide), dimethylaluminum(tri-phenylmethoxide), dimethylaluminum(triphenylsiloxide) and the like, dialkylaluminumhydride such as dimethylaluminumhydride, diisobutylaluminumhydride and the like.
Among these compounds, trialkylaluminum such as triisobutylaluminum is preferable.
In the present invention, an organoaluminum compound (C) may be used alone or in a combination of two or more.

Preparation of the catalyst of the present invention using the metallocene compound (A), the ionizing agent (B) and, if necessary, the organoaluminum compound (C), is preferably conducted under an inert gas atmosphere such as nitrogen.
The catalyst components used in the reaction may be prepared in advance in a catalyst preparation vessel, or alternatively, they may be prepared in a reactor used for the dimerization reaction of an α-olefin.
When the catalyst is prepared in the reactor, temperature is preferably adjusted to be lower than the temperature for the dimerization reaction of the α-olefin, for example, in the range of -30 to 200°C, and preferably 0 to 150°C.
Hydrogen may be introduced into the metallocene compound (A), the ionizing agent (B) and, if necessary, the organoaluminum compound (C) from the beginning without using an inert gas such as nitrogen, or alternatively, may be introduced after the metallocene compound (A), the ionizing agent (B), and, if necessary, the organoaluminum compound (C) are mixed and contacted with the α-olefin.

The mixing ratio of the metallocene compound (A) and the ionizing agent (B) in terms of the component(A)/the component (b-1) [molar ratio] is 1/1 to 1/1000 and preferably 1/2 to 1/100, and in terms of the component (A)/the component (b-2) [molar ratio] is 1/0.5 to 1/100 and preferably 1/1 to 1/20.
If the ratio of the component (A)/the component (b-1) [molar ratio] is less than 1/1, the catalyst activity may not be realized, while if the ratio exceeds 1/1000, a high molecular weight polymer of the α-olefin may be formed and the yield of the desired unsaturated hydrocarbon compound may be reduced.
Further, if the ratio of the component (A)/the component (b-2) [molar ratio] is less than 1/0.5, the catalyst activity may not be realized, while exceeding 1/100 does not proportionally improve the catalyst activity.
Also, the mixing ratio of the component (b-1) and the component (b-2) to the component (A) in terms of the component (A)/[the component (b-1) + the component (b-2)] (molar ratio) is 1/0.5 to 1/1000, and preferably 1/1 to 1/100.
The mixing ratio of the organoaluminum compound in terms of the component (C)/the component (A) [molar ratio] is 0/1 to 10000/1, and preferably 0/1 to 1000/1.
If the molar ratio of the component (C)/the component (A) exceeds 10000/1, a high molecular weight α-olefin polymer or a saturated dimer of the α-olefin may be formed and the yield of the desired unsaturated hydrocarbon compound may be reduced.

Although the amount of added hydrogen depends on the combination of the metallocene compound (A), the ionizing agent (B) and the organoaluminum compound (C), 0.1 to 700 kPaG, and preferably 0.5 to 100 kPaG.
Namely, the molar ratio of hydrogen to the metallocene compound (A) is 1/1 to 10000/1, preferably 1/1 to 1000/1, and more preferably 5/1 to 1000/1.
The molar ratio of hydrogen to the α-olefin is 1/10000 to 1/1, preferably 1/10000 to 1/10, and more preferably 1/2000 to 1/10.
If the amount of added hydrogen is not enough, the catalyst activity does not improve, if it is excessive, a saturated dimer of the α-olefin is formed and the yield of the desired unsaturated hydrocarbon compound may be reduced.

Although the α-olefin used in the present invention is not particularly limited, examples of preferable α-olefin include propylene, 1-butene, 3-methyl-1-butene, 4-methyl-1-butene, 4-phenyl-1-butcne, 1-pentene, 3-methyl-1-pentene, 4-methyl-1-pentene, 3,3-dimethyl-1-pentene, 3,4-dimethyl-1-pentene, 4,4-dimethyl-1-pentene, 1-hexene, 4-methyl-1-hexene, 5-methyl-1-hexene, 6-phenyl-1-hexene, 1-octene, 1-decene, 1-dodecene, 1-tetradecene, 1-hexadecene, 1-octadecene, I-eicocene, 1-dococene, 1-tetracocene, 1-hexacocene, 1-octacocene, 1-triacontene, 1-dotriacontene, 1-tetracontene, vinyl cyclohexane and the like.
In the present invention, the above α-olefins may be used alone or in a combination of two or more.
The unsaturated hydrocarbon compound obtained by the present invention normally contains 80 mol% or more of the unsaturated double bond, particularly the ratio of the terminal vinylidene group is normally 55 mol% or more.

In the present invention, the method for reaction is not limited, and the reaction may be conducted in the absence of a solvent or in the presence of a solvent, and either method may be employed.
With respect to the reaction conditions, the reaction temperature is -100 to 250°C, particularly -50 to 100°C is preferable.
The ratio of the amount of catalyst to α-olefin in terms of the α-olefin/the metallocene compound (A) (molar ratio) is normally 1000000/1 to 1000/1, and preferably 100000/1 to 2000/1.
The reaction time is normally 10 minutes to 48 hours.

If a solvent is used in the reaction, examples of the solvent include aromatic hydrocarbon such as benzene, toluene, xylene, ethylbenzene and the like, alicyclic hydrocarbon such as cyclopentane, cyclohexane, methyl cyclohexane and the like, aliphatic hydrocarbon such as pentane, hexane, heptane, octane and the like, halogenated hydrocarbon such as chloroform, dichloromethane and the like.
The above solvent may be used alone or in a combination of two or more.
Moreover, the raw material such as the α-olefin may be used as a solvent.

### Examples

The present invention is explained in further detail with reference to examples. However, the present invention is by no means restricted by these examples.

### Example 1

After 18 ml of toluene and 20 ml of 1-decene were charged in a glass container having an internal volume of 300 ml under atmospheric pressure at 25°C, 1.0 ml of a toluene solution of methylaluminoxane adjusted to 0.20 mol/L was added.
Subsequently, 20 ml of hydrogen (about 7 kPa) was introduced at 25°C, and 1.0 ml of a toluene solution of bis(cyclopentadienyl)zirconium dichloride adjusted to 20 mmol/L was added, and the reaction was carried out by heating up to 50°C while stirring for 5 hours.
The reaction was terminated by adding dilute hydrochloric acid, and the solution obtained after decomposing and removing the catalyst was analyzed by gas chromatography. The results indicated that the conversion of the raw material 1-decene was 70.0 mol%, and the yield of the dimer (unsaturated hydrocarbon compound) was 57.8 mol%.
Moreover, an analysis by ¹H-NMR indicated that the dimer contained 99.8 mol% of the unsaturated double bond (the ratio of 2-octyldodecene, namely, vinylidene group was 94.3 mol%).
The results are shown in Table 1.

### Example 2

A dimer (unsaturated hydrocarbon compound) was obtained by the same manner as in Example 1 except that 40 ml (about 13 kPa) of hydrogen was introduced.
The results indicated that the conversion of the raw material 1-decene was 72.7 mol%, the yield of the dimer was 55.0 mol%, and an analysis by ¹H-NMR indicated that the dimer contained 99.6 mol% of the unsaturated double bond (the ratio of vinylidene group was 95.5 mol%).
The results are shown in Table 1.

### Example 3

After 400 ml of 1-decene was charged in a stainless steel autoclave having an internal volume of 1000 ml under atmospheric pressure at 25°C, 20 ml of a toluene solution of methylaluminoxane adjusted to 0.10 mol/L was added.
Subsequently, 20 ml of a toluene solution of bis(cyclopentadienyl)zirconium dichloride adjusted to 10 mmol/L was added under atmospheric pressure at 25°C, followed by heating up to 50°C, and the reaction was carried out at 50°C for 5 hours under stirring and continuous introduction of hydrogen at 100 kPa.
The reaction was terminated by adding dilute hydrochloric acid, and the solution obtained after decomposing and removing the catalyst was analyzed by gas chromatography. The results indicated that the conversion of the raw material 1-decene was 100 mol%, and the yield of the dimer was 77.2 mol%.
Moreover, an analysis by ¹H-NMR indicated that the dimer contained 96.7 mol% of the unsaturated double bond (the ratio of vinylidene group was 92.7 mol%).
The results are shown in Table 1.

### Comparative Example 1

A dimer (unsaturated hydrocarbon compound) was obtained by the same manner as in Example 1, except that hydrogen was not introduced.
The results indicated that the conversion of the raw material 1-decene was 54.9 mol%, the yield of the dimer was 33.4 mol%, and an analysis by ¹H-NMR indicated that the dimer contained 99.8 mol% of the unsaturated double bond (the ratio of vinylidene group was 95.3 mol%).
The results are shown in Table 1.

### Example 4

After 19.25 of toluene and 20 ml of 1-decene were charged in a glass container having an internal volume of 300 ml under atmospheric pressure at 25°C, 0.25 ml of a toluene solution of triisobutyl aluminum adjusted to 1.0 mol/L, and 0.25 ml of a toluene solution of bis(pentamethylcyclopentadienyl)zirconium dichloride adjusted to 10 mmol/L of were added in this order.
Subsequently, 20 ml of hydrogen (about 7 kPa) was introduced at 25°C, and dimethylaniliniumtetrakis(pentafluorophenyl)borate suspended in 0.25 ml of toluene and adjusted to 10 mmol/L was added, followed by heating up to 100°C under stirring, and the reaction was carried out for 5 hours.
The reaction was terminated by adding dilute hydrochloric acid, and the solution obtained after decomposing and removing the catalyst was analyzed by gas chromatography. The results indicated that the conversion of the raw material 1-decene was 55.5 mol%, and the yield of the dimer was 24.4 mol%.
Moreover, an analysis by ¹H-NMR indicated that the dimer contained 98.5 mol% of the unsaturated double bond (the ratio of vinylidene group was 94.8 mol%).
The results are shown in Table 1.

### Example 5

After 400 ml of 1-decene was charged in a stainless steel autoclave having an internal volume of 1000 ml under atmospheric pressure at 25°C, 5.0 ml of a toluene solution of triisobutylaluminum adjusted to 1.0 mol/L was added.
Subsequently, 20 ml of a toluene solution of bis(pentamethylcyclopentadienyl)zirconium dichloride adjusted to 2.5 mmol/L and 10mmol/L of dimethylaniliniumtetrakis(pentafluorophenyl) borate suspended in 5 ml of toluene were added at 25°C, and heated up to 100°C.
The reaction was carried out at 100°C for 5 hours under stirring and continuous introduction of hydrogen at 100 kPa.
The reaction was terminated by adding dilute hydrochloric acid, and the solution obtained after decomposing and removing the catalyst was analyzed by gas chromatography. The results indicated that the conversion of the raw material 1-decene was 100 mol%, and the yield of the dimer was 23.9 mol%.
Moreover, an analysis by ¹H-NMR indicated that the dimer contained 81.5 mol% of the unsaturated double bond (the ratio of vinylidene group was 75.7 mol%).
The results are shown in Table 1.

### Comparative Example 2

A dimer (unsaturated hydrocarbon compound) was obtained by the same manner as in Example 3, except that hydrogen was not introduced.
The results indicated that the conversion of the raw material 1-decene was 48.7 mol%, the yield of the dimer was 20.8 mol%, and an analysis by ¹H-NMR indicated that the dimer contained 99.4 mol% of the unsaturated double bond (the ratio of vinylidene group was 95.7 mol%).
The results are shown in Table 1.

### Example 6

After 19.25 ml of toluene and 20 ml of 1-decene were charged in a glass container having an internal volume of 300 ml under atmospheric pressure at 25°C, 0.25 ml of a toluene solution of triisobutylaluminum adjusted to 1.0 mol/L and 0.25 ml of a toluene solution of bis(cyclopentadienyl)zirconium dichloride adjusted to 10 mmol/L were added.
Subsequently, 150 ml of hydrogen (about 50 kPa) was introduced at 25°C, and dimethylaniliniumtetrakis(pentafluorophenyl)borate suspended in 0.25 ml of toluene and adjusted to 10 mmol/L was added, followed by heating up to 100°C under stirring, and the reaction was carried out for 5 hours.
The reaction was terminated by adding dilute hydrochloric acid, and the solution obtained after decomposing and removing the catalyst was analyzed by gas chromatography. The results indicated that the conversion of the raw material 1-decene was 91.4 mol% and the yield of the dimer was 53.1 mol%.
Moreover, an analysis by ¹H-NMR indicated that the dimer contained 91 mol% of the unsaturated double bond (the ratio of vinylidene group was 56 mol%).
The results are shown in Table 1.

### Comparable Example 3

A dimer (unsaturated hydrocarbon compound) was obtained by the same manner as in Example 4, except that hydrogen was not introduced.
The results indicated that the conversion of the raw material 1-decene was 66.9 mol%, the yield of the dimer was 24.7 mol%, and an analysis by ¹H-NMR indicated that the dimer contained 95 mol% of the unsaturated double bond (the ratio of vinylidene group was 57 mol%).
The results are shown in Table 1.

[Table 1]

**Table 1**

| | Component (A) | Component (B) | Component (C) | Hydrogen (kPa) | Temperature (°C) | Time (hr) | Dimerization activity kg/g Zr-hr | Unsaturated double bond ratio (%) |
|---|---|---|---|---|---|---|---|---|
| Example 1 | 1a | 1b | | ca 7(*) | 50 | 5 | 0.95 | 99.8 |
| Example 2 | 1a | 1b | | ca 14(*) | 50 | 5 | 0.90 | 99.6 |
| Example 3 | 1a | 1b | | 100 | 50 | 5 | 2.54 | 96.7 |
| Comparative example 1 | 1a | 1b | | | 50 | 5 | 0.55 | 99.8 |
| Example 4 | 2a | 2b | 1c | ca 7(*) | 100 | 5 | 0.40 | 98.5 |
| Example 5 | 2a | 2b | 1c | 100 | 100 | 5 | 3.14 | 81.5 |
| Comparative example 2 | 2a | 2b | 1c | | 100 | 5 | 0.34 | 99.4 |
| Example 6 | 1a | 2b | 1c | ca 50(*) | 100 | 5 | 0.87 | 91.0 |
| Comparative example 3 | 1a | 2b | 1c | | 100 | 5 | 0.41 | 95.0 |

| | | | | | | | | |
|---|---|---|---|---|---|---|---|---|
| 1a : bis(cyclopentadienyl)zirconium dichloride 2a: bis(pentamethylcyclopentadienyl)zirconium dichloride 1b: methylalminoxane 2b: dimethylaniliniumtetrakis(pentafluorophenyl)borate 1c: triisobutylaluminum (*): initial pressure | | | | | | | | |

### [Industrial Applicability]

According to the present invention, there is produced an unsaturated hydrocarbon compound useful as a lubricant, cleaning agent, additive, its intermediate or the like, particularly the one having a terminal vinylidene group, in a high yield and high selectivity.

## Claims

1. A method for producing an unsaturated hydrocarbon compound, wherein an α-olefin is dimerized by using a catalyst composed of a metallocene compound (A) and an ionizing agent (B) in the presence of hydrogen.

2. The method for producing an unsaturated hydrocarbon compound according to claim 1, wherein the catalyst contains an organoaluminum compound (C) in addition to the component (A) and component (B).

3. The method for producing an unsaturated hydrocarbon compound according to claim 1 or 2, wherein the ionizing agent (B) is an oxygen-containing organometallic compound (b-1).

4. The method for producing an unsaturated hydrocarbon compound according to claim 3, wherein that component (b-1) is represented by the general formula (2) and/or the general formula (3), 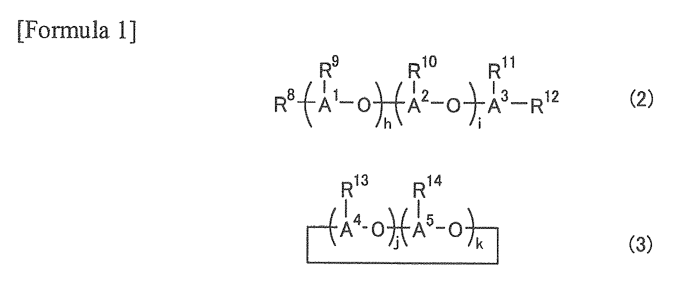 in the formula, R⁸ to R¹⁴ each independently represent an alkyl group having 1 to 8 carbon atoms, A¹ to A⁵ each independently represent a metal element of Group 13 in the periodic table, each of h to k is a number of 0 to 50, and both (h + i) and (j + k) are larger than 1.

5. The method for producing an unsaturated hydrocarbon compound according to claim 4, wherein A¹ to A⁵ are aluminum.

6. The method for producing an unsaturated hydrocarbon compound according to claim 1 or 2, wherein the ionizing agent (B) is a compound (b-2) forming an ionic complex by reacting with a metallocene compound (A).

7. The method for producing an unsaturated hydrocarbon compound according to claim 6, wherein the compound (b-2) is a coordination complex composed of an anion and a cation wherein plural groups are bonded to a metal and/ or a Lewis acid.

8. A method for producing an unsaturated hydrocarbon compound according to claim 7, wherein the coordination complex composed of an anion and a cation wherein plural groups are bonded to the metal is represented by the general formula (4) and/or (5),
([L¹ - H]^{g+})_{f}([M²D¹D² ••• Dⁿ]^{(n-m)-})₁ (4)
([L²]^{g+})_{f}([M³D¹D² ··· Dⁿ]^{(n-m)-})1 (5)
in the formula, L¹ represents a Lewis base, L² represents M⁴, R¹⁵R¹⁶M⁵ or R¹⁷₃C, M² and M³ each represent a metal selected from Group 5 to Group 15 in the periodic table, M⁴ represents a metal selected from Group 1 and Group 8 to Group 12 in the periodic table, M⁵ represents a metal selected from Group 8 to Group 10 in the periodic table, D¹ to Dⁿ each represent a hydrogen atom, a dialkylamino group, an alkoxy group, an aryloxy group, an alkyl group having 1 to 20 carbon atoms, an aryl group having 6 to 20 carbon atoms, an alkylaryl group, an arylalkyl group, a substituted alkyl group, an organometalloid group or a halogen atom, R¹⁵ and R¹⁶ each represent a cyclopentadienyl group, a substituted cyclopentadienyl group, an indenyl group or a fluorenyl group, R¹⁷ represents an alkyl group having 1 to 20 carbon atoms, an aryl group having 6 to 20 carbon atoms or an alkylaryl group, m is the valence of M² and M³, and is an integer of 1 to 7, n is an integer of 2 to 8, g is the ionic valence of [L¹ - H] and [L²] and is an integer of 1 to 7, f is an integer of 1 or larger, and 1 is a value calculated from the formula [f×g/(n - m)].

9. The method for producing an unsaturated hydrocarbon compound according to claim 8, wherein M² and M³ represent boron.

10. The method for producing an unsaturated hydrocarbon compound according to any of claims 1 to 9, wherein said component (A) is a compound represented by the general formula (1),
Qₐ(C₅H_{5-a-b}R¹_{b})(C₅H_{5-a-c}R²_{c})M¹XY (1)
in the formula, Q represents a connecting group for crosslinking two conjugated 5-membered ring ligands (C₅H_{5-a-b}R¹_{b}) and (C₅H_{5-a-c}R²_{c}), R¹ and R² each represent a hydrocarbon group, a halogen atom, an alkoxy group, a silicon-containing hydrocarbon group, a phosphorus-containing hydrocarbon group, a nitrogen-containing hydrocarbon group or a boron-containing hydrocarbon group, and may be the same or different from each other when plural groups are present, or may form a ring structure by bonding with each other, a is 0, 1 or 2, b and c each are an integer of 0 to 5 when a = 0, an integer of 0 to 4 when a = 1 and an integer of 0 to 3 when a = 2, M¹ represents a transition metal of Group 4 in the periodic table, and X and Y each represent a covalent bond ligand or an ionic bond ligand and may be bonded with each other.

11. The method for producing an unsaturated hydrocarbon compound according to claim 10, wherein M¹ represents zirconium.

12. The method for producing an unsaturated hydrocarbon compound according to any of claims 1 to 11, wherein the ratio of unsaturated double bonds contained in said unsaturated hydrocarbon compound is 80 mol% or more.
